# EUROPEAN PATENT SPECIFICATION

(11) **EP 1 470 818 B1**
(45) Date of publication and mention of the grant of the patent: **26.07.2006**
(21) Application number: 03291024.2
(22) Date of filing: 25.04.2003
(51) Int. Cl.: A61K 31/5415, A61P 25/28, A61P 25/16, A61P 25/02

(54) **Use of piperazine phenothiazine derivatives in the manufacture of a medicament with neuroprotector and/or neurotrophic effects on cns and/or pns**
Verwendung von Piperazin- Phenothiazin- Derivaten zur Herstellung eines Arzneimittels mit neuroprotektiven und/oder neurotropischen Wirkungen auf das ZNS und/oder PNS
Utilisation de dérivés de phénothiazine pipérazine pour la préparation d'un médicament ayant des effets neuroprotecteurs et/ou neurotrophiques sur le SNC et/ou SNP

(43) Date of publication of application: 27.10.2004
(73) Proprietor: NEURO3D, 68100 Mulhouse (FR)
(72) Inventor: Appert-Colin, Aline, 67200 Strasbourg (FR); Callizot, Noelle, 67200 Strasbourg (FR)
(74) Representative: Hirsch & Associés

(56) References cited:
- EP-A- 0 615 749
- WO-A-00/24390
- WO-A-00/32175
- WO-A-01/78709
- WO-A-01/92240
- WO-A-02/22611
- WO-A-96/04915
- WO-A-99/07356
- WO-A-02/083656
- WO-A-03/062232
- WO-A-03/062388
- US-A- 3 320 245
- US-A- 3 320 249
- US-A- 6 057 373
- US-B1- 6 482 822
- HALE M S ET AL: "LOW DOSE PERPHENAZINE AND L DOPA CARBIDOPA THERAPY IN A PATIENT WITH PARKINSONISM AND A PSYCHOTIC ILLNESS" JOURNAL OF NERVOUS AND MENTAL DISEASE, vol. 168, no. 5, 1980, pages 312-314, XP008020514 ISSN: 0022-3018
- PERINI M ET AL: "BENEFIT OF FLUOPERAZINE IN DRUG-INDUCED HALLUCINATIONS DURING PARKINSON'S DISEASE MANAGEMENT" GAZZETTA MEDICA ITALIANA ARCHIVIO PER LE SCIENZE MEDICHE, vol. 148, no. 3, 1989, pages 101-104, XP008020513 ISSN: 0393-3660
- VERMA ANITA ET AL: "N-Methyl-D-aspartate receptor participation in Parkinson's disease, a neurodegenerative disorder." ANNALS OF THE NEW YORK ACADEMY OF SCIENCES, vol. 765, 1995, page 327 XP008020512 Second International Conference;Lake George, New York, USA; July 31-August 3, 1994, Clinical and experimental aspects. 1995 New York Academy of Sciences 2 East 63rd Street, New York, New York 10021, USA ISBN: 0-89766-946-0
- "THE MERCK INDEX" 2001 , MERCK & CO., INC. XP002254640 * page 3780 * No. 3783: Ethopropazine
- GOTTLIEB G L ET AL: "Depot neuroleptics in the treatment of behavioral disorders in patients with Alzheimer's disease." JOURNAL OF THE AMERICAN GERIATRICS SOCIETY. UNITED STATES JUL 1988, vol. 36, no. 7, July 1988 (1988-07), pages 619-621, XP008026221 ISSN: 0002-8614
- DAVIS J L ET AL: "PERIPHERAL DIABETIC NEUROPATHY TREATED WITH AMITRIPTYLINE AND FLUPHENAZINE" JOURNAL OF THE AMERICAN MEDICAL ASSOCIATION, vol. 238, no. 21, 1977, pages 2291-2292, XP008026211 ISSN: 0098-7484
- MOSLEY C A ET AL: "CONTROL OF DIABETIC NEUROPATHIC PAIN BY AMITRIPTYLINE AND FLUPHENAZINE IN MILD RENAL FAILURE" KIDNEY INTERNATIONAL, vol. 21, no. 1, 1982, page 155 XP008026206 MEETING OF THE AMERICAN SOCIETY OF NEPHROLOGY, WASHINGTON, D.C., USA, NOV. 22-24, 1981. KIDNEY INT. ISSN: 0085-2538
- GÓMEZ-P¹REZ F J ET AL: "Nortriptyline-fluphenazine vs. carbamazepine in the symptomatic treatment of diabetic neuropathy." ARCHIVES OF MEDICAL RESEARCH. MEXICO 1996 WINTER, vol. 27, no. 4, January 1996 (1996-01), pages 525-529, XP001176770 ISSN: 0188-4409
- GOMEZ-PEREZ F J ET AL: "NORTRIPTYLINE AND FLUPHENAZINE IN THE SYMPTOMATIC TREATMENT OF DIABETIC NEUROPATHY A DOUBLE-BLIND CROSS-OVER STUDY" PAIN, vol. 23, no. 4, 1985, pages 395-400, XP008026204 ISSN: 0304-3959
- BATTLA H ET AL: "Clinical trial of amitriptyline and fluphenazine in diabetic peripheral neuropathy." SOUTHERN MEDICAL JOURNAL. UNITED STATES APR 1981, vol. 74, no. 4, April 1981 (1981-04), pages 417-418, XP008026223 ISSN: 0038-4348

## Description

### FIELD OF THE INVENTION.

This invention relates to a new use of piperazine phenothiazine derivatives and their pharmaceutically acceptable salts or esters in the manufacture of a medicament with neuroprotector and/or neurotrophic effects on CNS and/or PNS for the treatment of amyotrophic lateral sclerosis (ALS).

### BACKGROUND OF THE INVENTION

These compounds are known as major tranquilizers and neuroleptic drugs for their effects as relieving schizophrenic agitation, and maniacal behaviour. More particularly the piperazine series which includes trifluoroperazine, prochlorperazine, flufenazine are the most potent phenothiazine antipsychotic compounds. Flufenazine is marketed under the tradename Moditen® for its neuroleptic therapeutical effects. Fluphenazine and its hydrochloride salt or enanthate or decanoate ester form exerts activity at various levels of the CNS as well as on peripheral organ systems, which accounts for its antipsychotic action and side effects. Indirect evidence indicates that the antipsychotic effects of phenothiazines are linked to their effect in blocking dopamine and other catecholamine receptor sites.

WO03/062232 discloses derivatives of thiazoles comprising phenothiazine radical used for the treatment of neurological diseases such as ALS.

US6482822 discloses derivatives of N-iminomethyl amine comprising a phenothiazine radical having an inhibitory activity on NO synthetase enzymes producing NO and/or an activity which traps the reactive oxygen species and may produce favourable effects in the treatment of pathologies where these chemical species are involved. ALS diseases are cited,

WO02/083656 discloses 5-membered heterocyclic derivatives comprising a phenothiazine radical having an inhibitory activity on monoamine oxidase or on lipid peroxidation or having a modulation activity on sodium channels and which may be used to treat ALS.

WO00/32175 discloses phenothiazine derivatives useful for cancer treatment and capable of interacting with expression of a mutant protein of the p53. Also they are useful for diseases associated with conformationally unstable or misfolded protein such as ALS diseases.

### SUMMARY OF THE INVENTION

Surprisingly, the applicant has found that piperazine phenothiazine derivatives and more particularly flufenazine, are able to exert significant neuroprotective and neurotrophic effects. These new effects which could not be derived from actual flufenazine antipsychotic action have been highlighted during specific in vitro and in vivo model studies of CNS and PNS neuronal degeneration.

The invention relates to
1. Use of a compound of formula I 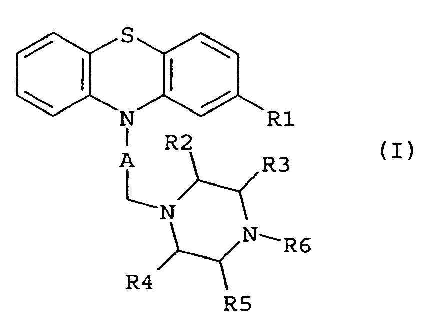 wherein
   A represents a straight or branched alkylene chain of from 2 to 6 carbon atoms separating the nitrogen atoms linked thereto by at least two carbon atoms ;
   R1 represents hydrogen, halogen, lower alkyl, lower alkoxy, lower alkanoyl, lower alkyl-mercapto, trifluoromethylmercapto, lower alkyl-sulfonyl (preferably methylsulfonyl), perfluoroalkyl of 1 to 3 carbon atoms;
   R2, R3, R4 and R5 each represent methyl, ethyl or hydrogen,
   R6 represents hydrogen, lower alkyl, hydroxy-lower-alkyl or aliphatic acyloxy-lower-alkyl having 1 to 4 carbon atoms in the acyloxy portion and 1 to 6 carbon atoms in the alkyl portion, CH₂-CH₂-O-R7 where R7 represents hydrogen, COR8 where R8 is a branched or straight chain alkyl radical of from seven to fourteen carbon atoms;
   in the manufacture of a medicament with neuroprotector and/or neurotrophic effects on CNS and/or PNS for the treatment of amyotrophic lateral sclerosis (ALS) diseases.
2 Use according to item 1 wherein
   A represents ethylene, propylene or 2- methylpropylene;
   R1 represents hydrogen, chloro, COCH₃, -CF₃;
   R2, R3, R4 and R5 each represent hydrogen;
   R6 represents CH₃, CH₂-CH₂-O-R7 where R7 represents hydrogen, COR8 where R8 is a straight chain alkyl radical of from seven to fourteen carbon atoms.
3. Use according to items 1 to 2, wherein the piperazine phenothiazine derivatives is flufenazine, or a pharmaceutically acceptable salt or ester thereof.
4. Use according to any one of items 1 to 3, wherein the medicament is for oral, rectal, subcutaneous, intramuscular or intravascular administration route.
5. Use according to any one of items 1 to 4, wherein the medicament comprises as active ingredient from 0,2mg to 500mg of the piperazine phenothiazine derivatives.
6. Use according to any one of items 1 to 5, wherein the medicament is to be administered at doses comprised between 0.1mg/kg to 10mg/kg.

The invention provides medicaments for the treatment amyotrophic lateral sclerosis (ALS) diseases. The above medicaments for the administration to human or other animal subjects comprise an effective amount of piperazine phenothiazine derivatives compounds of formula I having neuroprotector and/or neurotrophic effects.

### BRIEF DESCRIPTION OF THE FIGURES.

Fig 1: flufenazine effects on spinal cord neurons survival
Fig 2: protective effects on cortical neurons of flufenazine after glutamic acid intoxication and with maturation with BDNF.
Fig 3: protective effects of flufenazine on mesencephalic neurons after MPP+ intoxication
Fig 4: neurotrophic effects of flufenazine on cortical neurons
Fig 5: neurotrophic effects of flufenazine on spinal cord neurons
Fig 6: survival rate of SOD mice after oral administration of flufenazine.

### DETAILED DESCRIPTION

Thus, according to the present invention, there is provided the use of piperazine phenothiazine derivatives and a pharmaceutically acceptable salt and /or ester thereof, in the manufacture of a medicament with neuroprotector and neurotrophic effects on CNS and PNS for the treatment of amyotrophic lateral sclerosis (ALS)diseases

Piperazine phenothiazine derivatives are defined as compounds of the formula I 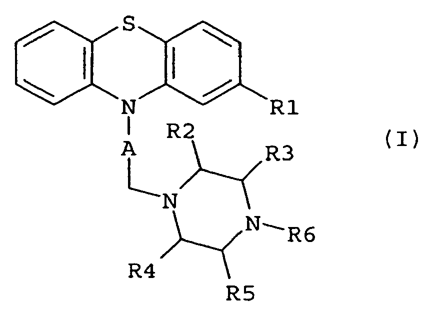 wherein
A represents a straight or branched alkylene chain of from 2 to 6 carbon atoms separating the nitrogen atoms linked thereto by at least two carbon atoms ;
R1 represents hydrogen, halogen (preferably chloro), lower alkyl, lower alkoxy, lower alkanoyl (preferably COCH₃), lower alkyl-mercapto, trifluoromethylmercapto, lower alkyl-sulfonyl (preferably methylsulfonyl), perfluoroalkyl of 1 to 3 carbon atoms, preferably CF₃ ;
R2, R3, R4 and R5 each represent methyl, ethyl or hydrogen;
R6 represents hydrogen, lower alkyl, hydroxy-lower-alkyl or aliphatic acyloxy-lower-alkyl having 1 to 4 carbon atoms in the acyloxy portion and 1 to 6 carbon atoms in the alkyl portion, CH₂-CH₂-O-R7 where R7 represents hydrogen, COR8 where R8 is a straight or branched chain alkyl radical of from seven to fourteen carbon atoms.

The terms "lower alkyl," "lower alkoxy", "lower alkanoyl" as employed herein include both straight and branched chain radicals of from 1 to 6 carbon atoms.

Preferably the piperazine phenothiazine derivatives used in the medicaments of the invention are selected from compounds of formula I wherein
A represents ethylene, propylene or 2-methylpropylene;
R1 represents hydrogen, chloro, COCH₃, CF₃;
R2, R3, R4 and R5 each represent hydrogen;
R6 represent CH₂-CH₂-O-R7 where R7 represents hydrogen, COR8 where R8 is a straight chain alkyl radical of from seven to fourteen carbon atoms.

More preferably, the piperazine phenothiazine derivatives used in the medicaments of the invention is 4-[3-[2-(trifluoromethyl)-10H-phenothiazin-10-yl]propyl]-1-pyperazineethanol (flufenazine) or salts and /or ester thereof.
These compounds and their chemical preparations under free bases form are disclosed in GB 829246, US 3.058.979.
This invention also includes salts of the above defined bases formed with non-toxic organic and inorganic acids.
Such salts are easily prepared by methods known in the art and are disclosed in GB 829246, US 3.058.979.
The invention also covers ester derivatives of the above compounds and their preparations are described in GB 833474 and US 3.194.733.

The medicaments of the invention are for treating amyotrophic lateral sclerosis (ALS)diseases.
The neuroprotective and neurotrophic effects of flufenazine were tested in vitro on primary neuronal cell cultures and in vivo, in animal model studies.

In vitro studies were first conducted on spinal cord motoneurons which are involved in peripheral neuropathy diseases as for example amyotrophic lateral sclerose (ALS) according to protocols described by Martinou J.C., Martinou I., Kato A.C. in Cholinergic differentiation factor (CDF/LIF) promotes survival of isolated rat embryonic motoneurons in vitro. Neuron 1992, 8(4) : 737-744) and by Ometani A, Nomoto H, Nitta A, Furukawa Y, Furukawa S. in 4-Methylcatechol stimulates phosphorylation of Trk family neurotrophin receptors and MAP kinases in cultured rat cortical neuron. J Neurosci Res 2002 Nov 1;70(3):335-9.

Further studies were conducted on cortical neurons intoxicated with glutamic acid according to the protocol described by Nilsen J. and Brinton RD in Impact of progestins on oestrogen-induced neuroprotection : synergy by progesterone and 19-norprogesterone and antagonism by medoxyprogesterone acetate. Endocrinology 143 : 205-212 2002.
Cortical neurons are involved in Alzheimer's disease and also in mesencephale neurons or dopaminergic neurons which are themselves involved in Parkinson's disease. (see also protocol described by Y. Mitsumotol, A. Watanabe, T. Miyauchi, F. Jimma, and T. Moriizumi in Stimulation of the regrowth of MPP+/damaged dopaminergic fibers by the treatment of mesencephalic cultures with basigin ; J Neural Transm (2001) 108: 1127-1134).
In vivo studies were conducted on transgenic animals with ALS causing mutations, a model for neurodegenerative diseases according to protocols described by Gurney Me, Pu H, Chiu Ay, Dal Canto Mc, Polchow Cy, Alexander Dd, Caliendo J, Hentati A, Kwon Yw, Deng Hx, et al. in Motor neuron degeneration in mice that express a human Cu ,Zn superoxide dismutase mutation. Science (1994) 264 : 1772-1775; and by Mohajeri Mh, Figlewicz Da, Bohn Mc in Selective loss of alpha motoneurons innervating the medial gastrocnemius muscle in a model of amyotrophic lateral sclerosis. Exp. Neurol. (1998) 150 : 329-336.
All these tests demonstrate that neuronal survival increase in the presence of several concentrations of flufenazine compared to the control without flufenazine and that flufenazine induces neuroprotective action after different intoxications.
The neurotrophic effect i.e. the neurite outgrowth with flufenazine was investigated on both cortical and spinal cord neuronal cultures according to the protocol described by Lucius R, Sievers J. in Postnatal retinal ganglion cells in vitro: protection against reactive oxygen species (ROS)-induced axonal degeneration by cocultured astrocytes Brain Res 1996 Dec 16;743(1-2):56-62.
The results on neurite length as well as the percentage of cells with neurites quantified by careful microscopic inspection demonstrate the neurotrophic effect in the presence of several flufenazine concentrations compared to the control without flufenazine.

The in vivo test results demonstrate the improved animals survival with the administration of several doses of flufenazine compared to the control without flufenazine.

These medicaments can be administered by oral, rectal, subcutaneous, intramuscular or intravascular administration routes.
The medicaments according to the invention can be solids or liquids and be presented in the pharmaceutical forms commonly used in human medicine, such as for example, plain or sugar-coated tablets, gelatin capsules, granules, suppositories, injectable preparations, ointments, creams, gels; they are prepared according to the usual methods. The active ingredient(s) can be incorporated with the excipients usually used in these pharmaceutical compositions, such as talc, gum arabic, lactose, starch, magnesium stearate, cocoa butter, aqueous or non aqueous vehicles, fatty substances of animal or vegetable origin, paraffin derivatives, glycols, various wetting, dispersing or emulsifying agents, preservatives.
These compositions can in particular be presented in the form of a powder intended to be dissolved extemporaneously in an appropriate vehicle, for example apyrogenic sterile water.
The medicament can comprise as active ingredient from 0,2mg to 500mg of the piperazine phenothiazine derivatives.
The dose administered is variable according to the condition treated, the patient in question, the administration route and the product considered. It can be, for example, comprised between 0,01mg and 50mg per day by oral route in adults with flufenazine or also comprised between 0.1 mg and 10mg per day by intramuscular or intravenous route.

### Examples

In vitro studies:

### Cell cultures conditions:

Two types of primary neuronal cell cultures i.e. cortical and spinal cord neurons are isolated from 15-17 days old foetuses of female Wistar rats and cultured in neurobasal/B27 medium until cell differentiation.

### Neuroprotection essays;

All tests are conducted with their appropriate control.

### Example 1

Maintenance of neuronal survival and proliferation is a crucial process for the integrity of neurons. In the present study spinal cord motoneuron cell culture are used to assess whether or not flufenazine promote neuronal survival and proliferation.

### Neuronal survival and proliferation

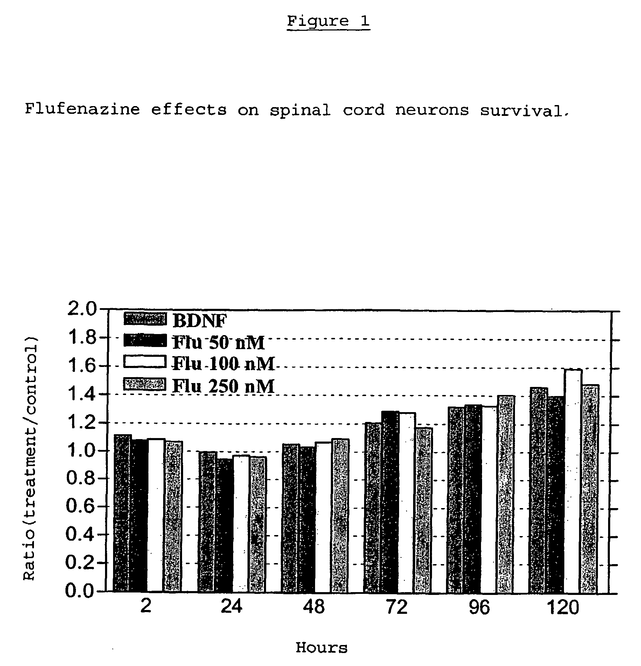

Cells cultures are incubated with different Fluphenazine N-Mustard dihydrochloride concentrations: 50, 100, 250 nmol/l.
Neuronal survival is then monitored at different time points; 2, 24, 48, 72, 96, 120 hours by counting the number of cells under microscope.
The results are depicted in Fig 1:
the percentage of neuronal survival obtained when cells cultures are incubated with flufenazine is compared to cell cultures incubated alone only with 50 microgram's of brain derived neurotrophic factor (BDNF). The whole percentages increase regularly from 48h until 120h and the best survival results are obtained with flufenazine concentrations of 50 to 250 nmol/l.

### Example 2

### Glutamic acid intoxication assay:

The neuroprotective activity is assessed on glu-induced cortical neurons loss. The process of death is initiated by 10 min treatment of neuron cell cultures with neurotoxic concentration of glutamic acid at 100 micromol/l.

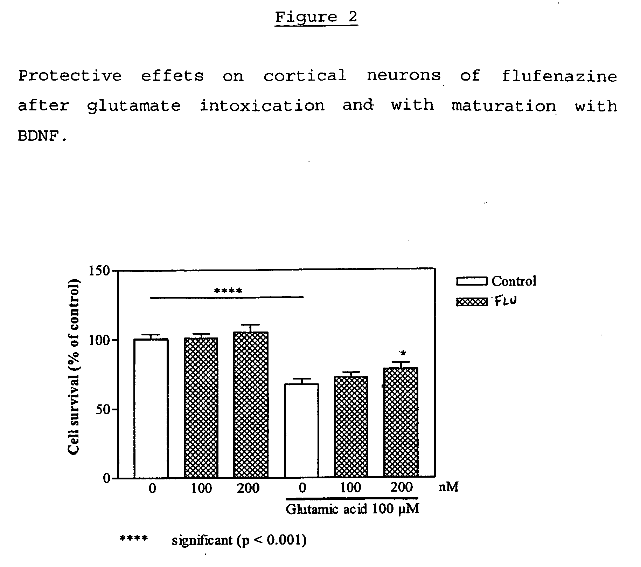

The ability of flufenazine to reverse the death process is achieved by subsequent exposure of cells to flufenazine concentrations of 100 and 200nmol/l. The quantity of LDH released is used to estimate the degree of intoxication and the decrease of quantity released is proportional at cellular resistance to Glu neurotoxicity. The results are depicted in Fig 2 (right part):
the percentage of neuronal survival obtained when intoxicated cells cultures are incubated with flufenazine is compared to cell cultures without flufenazine.
With 200nmol/l of flufenazine, a 10% increase is significantly observed.
In the left part of Fig 2, it can be observed that flufenazine has no or very slight effect, on cell survival in the absence of neurotoxic compound.

### Example 3

### MPP+ intoxication essay:

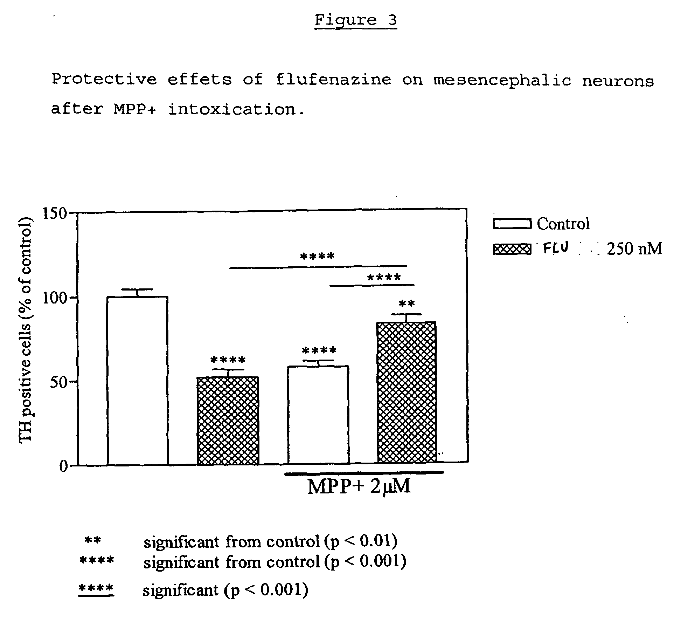

The neuroprotective activity is assessed on MPP+ induced mesencephalic neuron loss with flufenazine concentration of 250 nmol/l
The cells are intoxicated by 2 micromol of MPP+ as neurotoxic during 24h. The cell culture is then treated with 250nm/l of flufenazine. Reversed effects are observed after 48hours.
These neuroprotective effects are measured by the increased number of TH positive cells or dopaminergic neurons (i.e. mesencephale neurons which contains, tyrosine hydroxylase (TH), a dopamine synthesis enzyme.
The results are depicted in Fig 3 (right part):
The percentage of mesencephalic neuronal survival obtained when intoxicated cells cultures are incubated with flufenazine is compared to cell cultures without flufenazine.
With 250nmol/l of flufenazine, a 30% increase of TH positives cells is significantly observed.
These results show that flufenazine (250nmol/l) reverses MPP+ induced neuronal loss to the same extent as Riluzole (5 micromoles/l) a drug with established neuroprotective activity in this model (see reference Storch A, Burkhardt K, Ludolph AC, Schwarz J. Protective effects of riluzole on dopamine neurons: involvement of oxidative stress and cellular energy metabolism. J Neurochem 2000 Dec;75(6):2259-69).

In the left part of Fig 3, it can be confirmed that flufenazine according to its known extrapyramidal reactions has a negative effect (minus 50%) on the survival of this type of dopaminergic neurons.

In addition this assay also demonstrates longer neurite expansion than in control neurons at 250nmol/l).

### Example 4

### Neurotrophic assays:

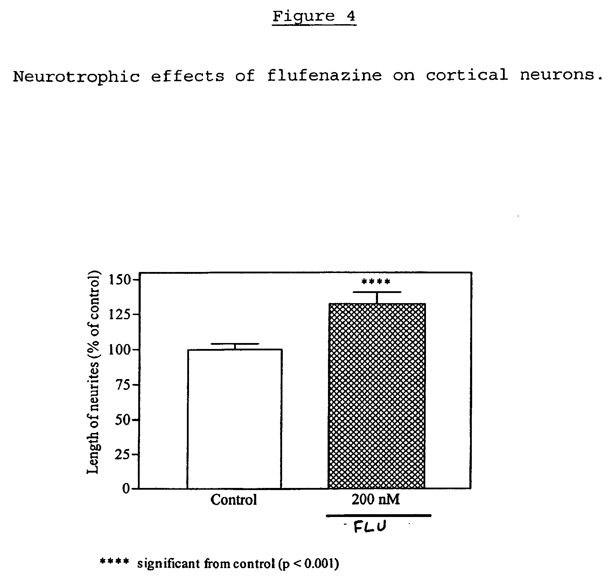

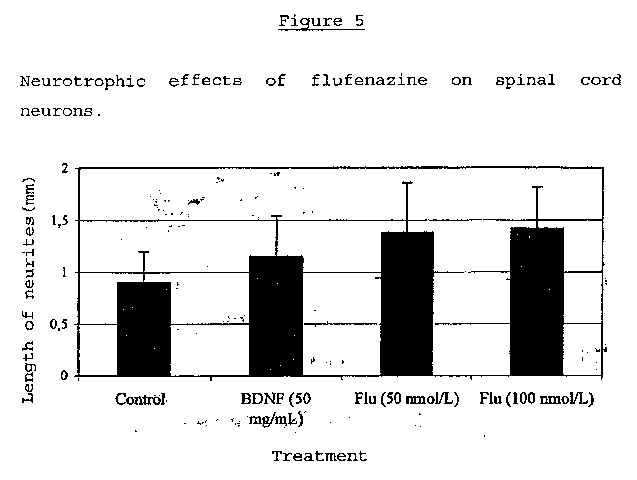

The ability of flufenazine to induce neurite outgrowth is investigated both in cortical and spinal cord neuronal cultures after 24h exposure to flufenazine.
The neurite length as well as the percentage of cells with neurites are quantified by careful microscopic inspection.
On cortex neurons type, the results are depicted in Fig 4; the cortex neurotrophic effect of flufenazine (200nmol/l) is expressed as an increase of 30% on neurites length compared to the neurites length in the control cortex neurons
On spinal cord neurons, the results are depicted in Fig 5:
The neurotrophic effect of flufenazine (50nmol/l, 100nmol/l) is expressed as an increase of 40 to 50% on neurites length compared to the neurites length in the control spinal cord neurons.

### Example 5

In vivo assays.

### Animals and flufenazine treatment.

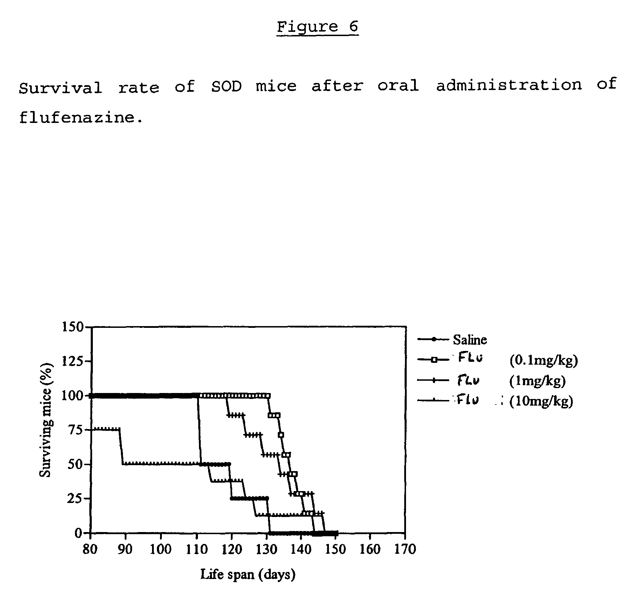

Mice are used at 4 months of age i.e. about 2 weeks before the appearance of the first symptoms.
They are genotyped for SOD1 gene using PCR method.
Animals are assigned into four groups to receive a daily oral administration of 1)saline as control, 2) 0,1mg/kg, 3) 1mg/kg, 4) 10mg/kg of flufenazine , until death by weakness and paralysis occurs.
Survival rate was recorded on a daily basis.
The results are depicted in Fig 6:
Doses of 0,1 and 1mg/kg flufenazine enhanced the survival of SOD mice as compared to that of the saline treated group. In contrast, higher dose (10mg/kg) appeared to have an adverse effect and induced a leftward shift in the survival curve.

## Claims

1. Use of a compound of general formula I 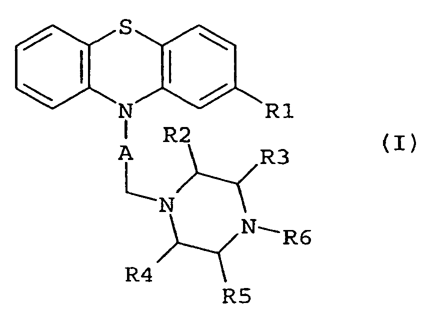 wherein
A represents a straight or branched alkylene chain of from 2 to 6 carbon atoms separating the nitrogen atoms linked thereto by at least two carbon atoms ;
R1 represents hydrogen, halogen, lower alkyl, lower alkoxy, lower alkanoyl, lower alkyl-mercapto, trifluoromethylmercapto, lower alkyl-sulfonyl (preferably methylsulfonyl), perfluoroalkyl of 1 to 3 carbon atoms;
R2, R3, R4 and R5 each represent methyl, ethyl or hydrogen,
R6 represents hydrogen, lower alkyl, hydroxy-lower-alkyl or aliphatic acyloxy-lower-alkyl having 1 to 4 carbon atoms in the acyloxy portion and 1 to 6 carbon atoms in the alkyl portion, CH₂-CH₂-O-R7 where R7 represents hydrogen, COR8 where R8 is a branched or straight chain alkyl radical of from seven to fourteen carbon atoms; the "lower alkyl," "lower alkoxy", "lower alkanoyl" including both straight and branched chain radicals of from 1 to 6 carbon atoms;
or flufenazine N-mustard dihydrochloride;
in the manufacture of a medicament with neuroprotector and/or neurotrophic effects on CNS and/or PNS for the treatment of amyotrophic lateral sclerosis (ALS)diseases.

2. Use according to claim 1 wherein
A represents ethylene, propylene or 2- methylpropylene;
R1 represents hydrogen, chloro, COCH₃, -CF₃;
R2, R3, R4 and R5 each represent hydrogen;
R6 represents CH₃, CH₂-CH₂-O-R7 where R7 represents hydrogen, COR8 where R8 is a straight chain alkyl radical of from seven to fourteen carbon atoms.

3. Use according to claims 1 to 2, wherein the compound of general formula I is flufenazine, or a pharmaceutically acceptable salt or ester thereof.

4. Use according to any one of claims 1 to 3, wherein the medicament is for oral, rectal, subcutaneous, intramuscular or intravascular administration route.

5. Use according to any one of claims 1 to 4, wherein the medicament comprises as active ingredient from 0,2mg to 500mg of the compound of general formula I.

6. Use according to any one of claims 1 to 5, wherein the medicament is to be administered at doses comprised between 0.1mg/kg to 10mg/kg.

## Patentansprüche

1. Verwendung einer Verbindung der allgemeinen Formel I 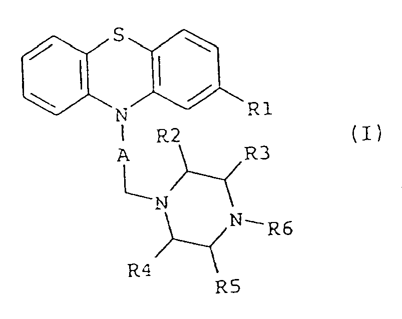 in der
A eine gerade oder verzweigte Alkylen-Kette von 2 bis 6 Kohlenstoffatomen, die die daran gebundenen Stickstoffatome durch mindestens 2 Kohlenstoffatome trennt, repräsentiert;
R1, Wasserstoff, Halogen, niedrigeres Alkyl, niedrigeres Alkoxy, niedrigeres Alkanoyl, niedrigeres Alky-mercapto, Trifluormethylmercapto, niedrigeres Alkyl-sulfonyl (bevorzugt mit Methylsulfonyl), Perfluoralkyl von 1 bis 3 Kohlenstoffatomen repräsentiert;
R2, R3, R4 und R5 jeweils Methyl, Ethyl oder Wasserstoff repräsentieren,
R6 Wasserstoff, niedrigeres Alkyl, Hydroxy-niedrigeres Alkyl oder aliphatisches Acyloxy-niedrigeres Alkyl mit 1 bis 4 Kohlenstoffatomen in dem Acyloxy-Teil und 1 bis 6 Kohlenstoffatomen in dem Alkyl-Teil, CH₂-CH₂-O-R7, worin R7 Wasserstoff repräsentiert, COR8, worin R8 eine verzweigte oder geradkettige Alkylgruppe von 7 bis 14 Kohlenstoffatomen ist, repräsentiert; wobei "niedrigeres Alkyl", "niedrigeres Alkoxy", "niedrigeres Alkanoyl" sowohl geradkettige als auch verzweigtkettige Gruppen von 1 bis 6 Kohlenstoffatomen umfaßt:
oder Flufenazin-N-lost-dihydrochlorid;
zur Herstellung eines Arzneimittels mit Neuroprotektor-Wirkungen und/oder neurotrophischen Wirkungen aus das ZNS und/oder PNS zur Behandlung amyotrophischer Lateralsklerose (ALS)-Krankheiten.

2. Verwendung nach Anspruch 1, bei der
A Ethylen, Propylen oder 2-Methylpropylen repräsentiert;
R1 Wasserstoff, Chlor, COCH₃, -CF₃ repräsentiert;
R2, R3, R4 und R5 jeweils Wasserstoff repräsentieren;
R6 CH₃, CH₂-CH₂-O-R7, worin R7 Wasserstoff repräsentiert, COR8, worin R8 eine geradkettige Alkyl-Gruppe von 7 bis 14 Kohlenstoffatomen ist, repräsentiert.

3. Verwendung nach den Ansprüchen 1 bis 2, bei der die Verbindung der allgemeinen Formel I Flufenazin oder ein pharmazeutisch annehmbares Salz oder ein pharmazeutisch annehmbarer Ester davon ist.

4. Verwendung nach einem der Ansprüche 1 bis 3, bei der das Arzneimittel für einen oralen, rektalen, subkutanen, intramuskulären oder intravaskulären Verabreichungsweg ist.

5. Verwendung nach einem der Ansprüche 1 bis 4, bei der das Arzneimittel als Wirkstoff von 0,2 mg bis 500 mg der Verbindung der allgemeinen Formel I aufweist.

6. Verwendung nach einem der Ansprüche 1 bis 5, bei der das Arzneimittel in Dosen zu verabreichen ist, die zwischen 0,1 mg/kg bis 10 mg/kg liegen.

## Revendications

1. Utilisation d'un composé de formule générale 1 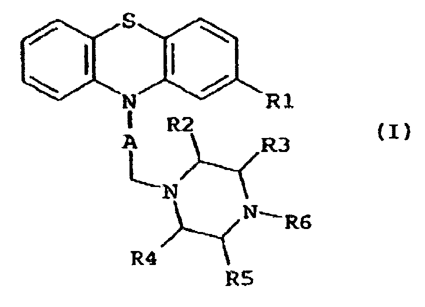 dans laquelle:
A représente une chaîne alkylène linéaire ou ramifiée de 2 à 6 atomes de carbone séparant les atomes d'azote liés à celui-ci par au moins deux atomes de carbone ;
R¹ représente un atome d'hydrogène, un atome d'halogène, un groupe alkyle inférieur, un groupe alcoxy inférieur, un groupe alcanoyle inférieur, un groupe (alkyle inférieur) mercapto, un groupe trifluorométhylmercapto, un groupe (alkyle inférieur)sulfonyle (de préférence le groupe méthylsulfonyle), un groupe perfluoroalkyle de 1 à 3 atomes de carbone ;
R², R³, R⁴ et R⁵ représentent chacun le méthyle, l'éthyle ou un atome d'hydrogène,
R⁶ représente un atome d'hydrogène, un groupe alkyle inférieur, un groupe hydroxy-alkyle inférieur ou un groupe acyloxy-alkyle inférieur aliphatique ayant de 1 à 4 atomes de carbone dans la partie acyloxy et de 1 à 6 atomes de carbone dans la partie alkyle, CH₂-CH₂-O-R⁷, où R⁷ représente un atome d'hydrogène, COR⁸ où R⁸ est un radical alkyle à une chaîne ramifiée ou linaire de 7 à 14 atomes de carbone ; les termes « alkyle inférieur », « alcoxy inférieur », « alcanoyle inférieur » incluant à la fois des radicaux à chaînes linéaire et ramifiée de 1 à 6 atomes de carbone ;
ou de dihydrochlorure de fluphénazine-N-mustard ;
pour la fabrication d'un médicament ayant des effets neuroprotecteurs et/ou neurotrophiques sur le SNC et/ou le SNP pour le traitement de scléroses latérales amyotrophiques (SLA).

2. Utilisation selon la revendication 1, dans laquelle
A représente l'éthylène, le propylène ou le 2-méthyl-propylène ;
R¹ représente un atome d'hydrogène, un atome de chlore, COCH₃, -CF₃ ;
R², R³, R⁴ et R⁵ représentent chacun un atome d'hydrogène ;
R⁶ représente CH₃, CH₂-CH₂-O-R⁷, où R⁷ représente un atome d'hydrogène, COR⁸ où R⁸ est un radial alkyle à une chaîne linaire de sept à quatorze atomes de carbone.

3. Utilisation selon les revendications 1 à 2, dans laquelle le composé de formule générale I est la fluphénazine ou un sel pharmaceutiquement acceptable ou un ester de celle-ci.

4. Utilisation selon l'une quelconque des revendications 1 à 3, dans laquelle le médicament est destiné une administration par voie orale, rectale, sous-cutanée, intramusculaire ou intraveineuse.

5. Utilisation selon l'une quelconque des revendications 1 à 4, dans laquelle le médicament comprend comme principe actif de 0,2 mg à 500 mg du composé de la formule générale I.

6. Utilisation selon l'une quelconque des revendications 1 à 5, dans laquelle le médicament est administré à des doses allant de 0,1 mg/kg à 10 mg/kg.
